# EUROPEAN PATENT SPECIFICATION

(11) **EP 1 268 409 B1**
(45) Date of publication and mention of the grant of the patent: **30.11.2005**
(21) Application number: 01927725.0
(22) Date of filing: 13.03.2001
(51) Int. Cl.: C07C 269/04, C07C 271/28

(54) **CONTINUOUS PROCESS FOR THE SYNTHESIS OF AROMATIC URETHANES**
KONTINUIERLICHES VERFAHREN ZUR HERSTELLUNG VON AROMATISCHEN URETHANEN
PROCEDE CONTINU DE SYNTHESE D'URETHANES AROMATIQUES

(30) Priority: 17.03.2000 IT MI000547
(43) Date of publication of application: 02.01.2003
(73) Proprietor: DOW GLOBAL TECHNOLOGIES INC., Midland, Michigan 48674 (US)
(72) Inventor: CESTI, Pietro, I-28069 Trecate-Novara (IT); BOSETTI, Aldo, I-13100 Vercelli (IT); BATTISTEL, Ezio, I-28060 Cameriano (IT); CARLETTI, Vittorio, I-28100 Novara (IT); BIGNAZZI, Renzo, I-20025 Legnano-Milan (IT)
(74) Representative: Raynor, John
(86) International application number: PCT/EP2001/002829
(87) International publication number: WO 2001/068590

(56) References cited:
- EP-A- 0 391 473
- EP-A- 0 881 213
- GB-A- 2 091 730

## Description

The present invention relates to a process for the preparation of aromatic urethanes which consists in reacting organic carbonates with aromatic amines, in reactors operating in continuous, capable of maintaining a suitable concentration of amine in the reaction mixture.

Aromatic urethanes are valuable intermediates used for the production of phyto-drugs, dyes, pharmaceutical compounds, and also aromatic isocyanates used in the synthesis of polyurethanes: among aromatic urethanes, those of greatest interest from an industrial point of view are 4,4'-methylenediphenyldiurethane (MDU) and toluenediurethane (TDU) used for the preparation of methylene-diphenyl diisocyanate (MDI) and toluene diisocyanate (TDI).

Processes are known for the production of urethanes based on the functionalization of amines with an organic carbonate, preferably dimethylcarbonate (DMC), in the presence of suitable catalysts according to the following reaction scheme:

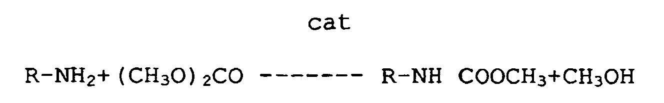

For example, Italian patent 1,141,960 describes a process for the preparation of aromatic urethanes by means of the reaction between aromatic amines and alkyl carbonates, said reaction taking place in the presence of a catalyst consisting of an alcoholate of an alkaline or earth-alkaline metal. The reaction is carried out in liquid phase in the presence of a molar ratio carbonate/amine varying from 10/1 to 1/1.

Italian patent 1,229,144 describes a process for the preparation of carbamates which comprises the initial reaction between alkyl or cyclo-alkyl carbonate, in a quantity equal to or higher than the stoichiometric value, and an amine, causing the formation of a mixture of carbamate and urea, and the subsequent reaction of the urea thus formed with carbonate: carbamation catalysts consisting of Lewis acids such as bivalent tin and zinc chlorides, or salts of said metals with mono-or bi-carboxylic organic acids, or again trivalent fluorine compounds such as iron chloride and acetylacetonate, are used for the purpose.

The processes for the preparation of aromatic urethanes according to the two patents mentioned above, are effected so as to minimize the formation of by-products typical of the reaction between organic carbonates and aromatic amines: ureas and N-methylated compounds. It is known in fact that ureas are formed by the reaction of an amine group, which has not yet reacted, with a urethane group of another molecule and which may however be converted to urethanes in a subsequent finishing phase, which in fact reduces the damage.

N-methylated products on the other hand, are extremely harmful as, in addition to reducing the yield to urethane in the synthesis, they significantly lower the yield in the subsequent thermal decomposition reaction of urethane to the corresponding isocyanate: in fact the N-methylate group is capable of reacting with the isocyanate already formed producing compounds of a pitchy nature.

According to the known art, of which the two above patents represent an example, it is therefore possible to limit the formation of these by-products by resorting to the use of particular catalytic systems, or by carrying out the process in different reactive steps.

The Applicant has now found that the formation of the above by-products, in particular N-methylated compounds, can be further reduced by effecting the synthesis of aromatic urethanes in continuous reactors allowing low concentrations of aromatic amine to be used.

Without any intention of explaining the process mechanisms, it has in fact been found that the formation of N-methylated products is influenced by the concentration of amine present in the reaction environment: when operating in batch reactors, into which all of the amine is charged in the initial phase, its concentration is high and this leads to the formation of significant quantities of N-methylated by-products; when operating, on the contrary, in suitable continuous reactors, it is possible to keep a low concentration of the amine in the reaction environment and reduce the formation of N-methylated compounds to well below 1% of the total reacted amine groups.

An object of the present invention according to the present patent application therefore relates to a process for the preparation of aromatic urethanes which consists in reacting, in continuous, organic carbonates with aromatic amines maintaining low concentrations of the latter. Reactors suitable for operating in continuous can be advantageously used for the purpose, such as for example reactors of the CSTR type, single or in series, or of the reactive column type.

The aromatic amines which can be used are represented by formula (I):

R- (NH₂)ₙ (I)

wherein n is an integer from 1 to 2, R represents an aryl radical, such as monovalent, bivalent radicals of benzene, toluene, naphthalene, diphenyl, methylene-diphenyl.

The aryl radical may contain, as substituents, atoms or radicals non-reactive with the isocyanate function, such as halogen atoms, alkoxy, nitro, cyano, acyl, acyloxy, isocyanate groups.

Non-limiting examples of aromatic amines having formula (I) are: 2,4-diaminotoluene, 2,6-diaminotoluene or mixtures of the two isomers, aniline, toluidine, 3,5-dichloroaniline, 4,4'-methylenedianiline, 2,4'-methyle-nedianiline, 2,2'-methylenedianiline or mixtures of isomers.

Catalysts suitable for the purposes of the present invention generally consist of Lewis acids such as salts of tin, copper and zinc, or salts of said metals with mono- or bi-carboxylic organic acids. These salts have a pka equal to or higher than 2.8. Among zinc compounds, anhydrous acetate or dihydrate is preferably used.

The reactions comprise the use of organic carbonate as reagent and solvent at the same time and the removal of the alcohol by-product as it forms; the total quantity of aromatic amine which is fed in continuous to the reaction ranges from 4 to 30% by weight with respect to the total mixture. According to the present invention, the reaction is carried out in suitable reactors, operating in continuous in order to maintain the concentration of the aromatic amine preferably within a value ranging from 0.1 to 5% by weight with respect to the total mixture, and, in particular, from 0.1 to 2.5% by weight, so as to minimize the formation of by-products of an N-methylated nature. The excess carbonate used in the reaction is separated from the raw product, according to methods well known to experts in the field.

The process according to the present invention is now described in greater detail with reference to the synthesis of toluenediurethane (TDU), owing to the great industrial interest in this compound: this example is naturally only provided for illustrative purposes and in no way limits the scope of the present invention.

The following indications will provide experts in the field with the necessary disclosures for the preparation of a wide range of urethanes.

### EXAMPLE 1: CSTR SYNTHESIS IN SERIES

With respect to the continuous synthesis of TDU in CSTR-type reactors, the transformation reaction of toluenediamine (TDA 80/20, mixture of 2,4/2,6 isomers in a proportion of 80/20) into the corresponding mixture of urethanes can be effected in a continuous plant, for example consisting of 4 CSTR-type reactors in series, each of which having a useful volume of 0.5 liters, in which the amine is transformed into a mixture of TDU (toluenediurethane)/TMU (toluenemonourethane)/ureas and a final 5-liter reactor in which the complete conversion to TDU is effected.

Each CSTR is equipped with a heating jacket, differential pressure level control, removal system of the methanol/DMC vapor phase and a reintegration system of the vaporized DMC and the reactors are connected to each other by means of a line equipped with a level control regulated valve.

The CSTRs are initially pressurized with nitrogen, by means of a suitable pressure regulation valve system. During the reaction, the pressure of the vapor phase at the head of each single CSTR is kept constant by means of automatic regulation flow valves.

The plant is completed by automatic sampling systems at the outlet of each CSTR, a collection tank of the evaporated phase after condensation and a feeding tank of the reagent mixture to the first CSTR. All the process lines are electrically heated and insulated to avoid any possible precipitations of the mixtures of products.

The internal temperature of the reagent mixture is kept constant at 160°C, and the pressure at 6 ata. The temperature, preselected at a certain value, is kept constant for the whole duration of the test.

The mixture of TDU (toluenediurethane)/TMU (toluenemonourethane)/ureas leaving the CSTR is finally sent to a stirred reactor (finisher) having a volume of 5 1, thus capable of allowing residence times of about 2-3 hours, and is transformed to TDU. The reactor is kept at a temperature ranging from 160 to 180°C, preferably about 170°C. The pressure is maintained at values not exceeding 9 ata.

Using the system described above, a test was effected, continuously removing the methanol/DMC phase in each single CSTR.

An 80/20 mixture of TDA (equal to 88 g/L in DMC) and 4.7 g/L of zinc acetate dihydrate (equal to 3% moles of catalyst per mole of TDA) in DMC, is fed to the first CSTR.

The residence time in each CSTR is 15 minutes.

A sample is taken from the 1^{st} CSTR, under regime conditions, determining from HPLC analysis a 51.8% conversion of the amine groups to products such as TDU, monourethanes and ureas.

The concentration of non-reacted TDA proved to be equal to 11.4 g/L or equal to 12.9% of the initial TDA. The N-methylated products present in the reaction are equal to 1.5% of the converted amine groups. This value corresponds to a ratio of N-methylated compounds with respect to the total converted compounds equal to 0.9.

The reaction mixture, leaving the 4^{th} CSTR is subsequently sent to the finisher and maintained at 170°C for 3 hours to give an 80/20 TDU product having a chemical purity equal to 95%.

The percentage of N-methylated products proved to be equal to 0.7°s of the converted amine groups.

### EXAMPLE 2: SYNTHESIS IN CSTR IN SERIES

Using the same system described above, a test was effected operating in the presence of a removal of the methanol/DMC phase in each single CSTR.

An 80/20 mixture of TDA (equal to 60 g/L in DMC) and 3.24 g/L of zinc acetate dihydrate (equal to 3% moles of catalyst per mole of TDA) in DMC, is fed to the first CSTR.

The residence time in each CSTR is 15 minutes.

A sample is taken from the 1^{st} CSTR under regime conditions, determining from HPLC analysis a 61.4% conversion of the amine groups to products such as TDU, monourethanes and ureas.

The concentration of non-reacted TDA proved to be equal to 5.12 g/L or equal to 8.53% of the initial TDA. This value corresponds to a ratio of N-methylated compounds with respect to the total converted compounds equal to 0.65.

The reaction mixture is subsequently sent to the finisher and maintained at 170°C for 3 hours to give an 80/20 TDU product having a chemical purity equal to 95.5%.

The percentage of N-methylated products proved to be equal to 0.55% of the converted amine groups.

### EXAMPLE 3: PLANT DESCRIPTION FOR THE CONTINUOUS SYNTHESIS OF TDU IN A REACTIVE COLUMN.

The reaction for the transformation of TDA 80/20 into the corresponding mixture of urethanes is carried out in a continuous plant.

The plant consists of a column into which the reaction mixture is fed from above and is put in contact in countercurrent with the DMC vapors rising from below, in order to create an equilibrium which allows the methanol to be removed from the reaction.

The liquid leaving the bottom of the column enters a reboiler in which DMC vapors are generated, by means of an external heat source, which are fed from the bottom of the column.

The amine is transformed in the liquid leaving the reboiler into a mixture of TDU (toluenediurethane)/TMU (toluenemonourethane)/ureas.

The complete conversion to TDU is effected in a final reactor situated after the reboiler.

The removal of the DMC/methanol vapor phase is effected at the head of the column by means of an automatic regulation flow valve which allows the reaction system to be maintained at the desired pressure.

The reboiler at the bottom of the column is equipped with a heat flow control system to check the quantity of DMC vapor produced.

The same reboiler, which also acts as reaction step, is equipped with automatic level control which activates the removal valve of the liquid phase.

The plant is completed by automatic sampling systems at the outlet of the reboiler, a collection tank of the evaporated phase after condensation, and a feeding tank of the reagent mixture. All the process lines are electrically heated and insulated to avoid any possible precipitations of the mixtures of products.

The internal temperature of the reagent mixture is set at 160°C, and the pressure at 6 ata.

The column consists of about 20 theoretical plates, allowing an overall residence time of the liquid phase of 1-3 hours.

The reboiler has a volume corresponding to a residence time of 30 minutes.

The mixture of TDU/TMU/ureas leaving the reboiler is finally sent to a reactor capable of allowing residence times of about 2-3 hours, and is transformed into TDU. The reactor is kept at a temperature ranging from 160 to 180°C, preferably 170°C. The pressure is maintained at values not exceeding 9 ata.

Using the system described above, a test was carried out feeding the column from above with a solution, preheated to 85°C, of TDA 80/20 (equal to 40 g/L in DMC) and 3.1 g/L of zinc acetate dihydrate (equal to 3% moles of catalyst per mole of TDA) in DMC.

The residence time for each single step is 6 minutes equal to a total of 2 hours whereas the mixture is kept for 30 minutes in the reboiler.

The distillation degree of the vapors in countercurrent inside the column is equal to 0.6 V distillate/V fed.

The mixture leaving the bottom of the column, consisting of TDU (toluenediurethane)/TMU (toluenemonourethane)/ ureas (about 190 g/L) is kept for 30 minutes in the boiler.

A sample is taken at the outlet of the reboiler under regime conditions, determining from HPLC analysis a 96.5% conversion of the amine groups to products such as TDU, monourethanes and ureas.

The TDA has completely disappeared. The N-methylated products present in the reactions are equal to 0.93% of the converted amine groups. This value corresponds to a ratio of N-methylated compounds with respect to the total converted compounds equal to 0.96%.

The reaction mixture is subsequently sent to the finisher and maintained at 170°C for 3 hours to give an 80/20 TDU product having a chemical purity equal to 96.5%.

The percentage of N-methylated products proved to be equal to 0.9% of the converted amine groups.

### COMPARATIVE EXAMPLE 4

165 g of TDA 80:20, 1600 g of DMC and 8.9 g of zinc acetate dihydrate (3% in moles with respect to the TDA) were charged under a nitrogen atmosphere into a cylindrical autoclave having a useful volume of 3 liters. The autoclave was then heated to 160°C for 1.5 hours under stirring (300 rpm), continuously removing the methanol/DMC mixture by distillation. After 1.5 hours of reaction, the distillation was interrupted and the temperature inside the reactor was brought to 170°C for 3 hours (finishing phase).

Upon HPLC analysis of the reaction, it was found that the conversion of the initial TDA was 99% with a selectivity to TDU equal to 94%. The N-methylated products corresponded to 3.2% with respect to the converted amine groups.

### COMPARATIVE EXAMPLE 5

400 ml of DMC and 75 g of TDA 80:20 were charged into a steel autoclave having a volume of 1000 ml. The autoclave was heated to 160°C under stirring (300 rpm) in a nitrogen atmosphere.

When the operating temperature had been reached, zinc acetate hydrate (3.94 g, equal to 3% in moles with respect to the TDA) in 100 ml of DMC was charged into the autoclave under pressure and heat. After 15 minutes a sample of the reaction mixture was taken. HPLC analysis showed that the non-reacted TDA was equal to 75% of the initial quantity, corresponding to a concentration of 87 g/L. The N-methylated products present in the reaction proved to be 12.5% with respect to the converted amine groups.

### COMPARATIVE EXAMPLE 6

400 ml of anhydrous DMC and 75 g of TDA 80:20 were charged into a steel autoclave having a volume of 1000 ml. After stabilization of the operating conditions (temperature 160°C, stirring at 300 rpm in an nitrogen atmosphere), 3.94 g of zinc acetate hydrate (3% in moles with respect to the TDA) in 100 ml of DMC were charged into the autoclave under pressure and heat. A sample of the reaction mixture was taken after 30 minutes. An analysis of the reaction products was carried out by means of HPLC. It was found that 88% of the TDA had reacted (remaining TDA equal to 12% of the initial TDA, corresponding to a concentration of 17.5 g/L). The quantity of N-methylated derivatives with respect to the converted amine groups was 5.2%.

## Claims

1. A process for the preparation of aromatic urethanes consisting in reacting organic carbonates with aromatic amines in reactors operating in continuous and feeding a total quantity of aromatic amines in a percentage with respect to the weight of the total mixture ranging from 4 to 30.

2. A process according to claim 1 for the preparation of aromatic urethanes wherein the reaction between organic carbonates and aromatic amines is carried out in reactors in continuous mode, the concentration of the aromatic amine being maintained at 0.1 to 5% by weight with respect to the total mixture.

3. A process according to claim 2, wherein the concentration of aromatic amine is preferably maintained at 0.1 to 2.5% with respect to the total mixture.

4. A process according to any one of the preceding claims wherein the reaction is carried out in a reactor selected from those of the CSTR (continuously stirred tank reactor) and reactive column type.

5. A process according to any one of the preceding claims wherein the reaction is carried out in the presence of a catalyst selected from Lewis acids with a pka equal to or higher than 2.8.

6. A process according to claim 5 wherein the reaction between organic carbonate and aromatic amine is carried out in the presence of anhydrous zinc acetate or zinc acetate dihydrate.

7. A process according to any one of the preceding claims wherein the reaction is carried out in the presence of organic carbonate alone acting as solvent.

8. A process according to any one of the preceding claims wherein the reaction is accompanied by the removal of the alcohol, as it is formed.

9. A process according to any one of the preceding claims wherein the reaction is carried out starting from an amine having the formula R- (NH₂)ₙ wherein n is 1 or 2, R represents an aryl radical selected from monovalent and bivalent radicals of benzene, toluene, napthalene, diphenyl, methylene-diphenyl.

10. A process according to any one of the preceding claims wherein the reaction is carried out starting from an amine selected from 2,4-diaminotoluene; 2,6-diaminotoluene; mixtures of 2,4-diaminotoluene and 2,6-diaminotoluene; aniline; toluidine; 3,5-diaminotoluene; 4,4-methylenedianiline; 2,4-methylenedianiline; 2,2-methylenedianiline; and mixtures of any 2 or more of 4,4-methylenedianiline, 2,4-methylenedianiline and 2,2-methylenedianiline.

## Patentansprüche

1. Verfahren zur Herstellung von aromatischen Urethanen, bestehend aus dem Reagieren von organischen Carbonaten mit aromatischen Aminen in kontinuierlich betriebenen Reaktoren und dem Zuführen der aromatischen Amine in einer Gesamtmenge im Bereich von 4 bis 30 Prozent, bezogen auf das Gewicht der Gesamtmischung.

2. Verfahren nach Anspruch 1 zur Herstellung von aromatischen Urethanen, wobei die Reaktion zwischen den organischen Carbonaten und den aromatischen Aminen in Reaktoren in einem kontinuierlichen Modus durchgeführt wird, wobei die Konzentration des aromatischen Amins bei 0,1 bis 5 Gew.-%, bezogen auf die Gesamtmischung, gehalten wird.

3. Verfahren nach Anspruch 2, wobei die Konzentration des aromatischen Amins vorzugsweise bei 0,1 bis 2,5 %, bezogen auf die Gesamtmischung, gehalten wird.

4. Verfahren nach einem der vorhergehenden Ansprüche, wobei die Reaktion in einem Reaktor, ausgewählt aus denjenigen vom CSTR (kontinuierlicher Rührkessel)-Typ und Reaktivkolonne-Typ, durchgeführt wird.

5. Verfahren nach einem der vorhergehenden Ansprüche, wobei die Reaktion in Gegenwart eines Katalysators, ausgewählt aus Lewis-Säuren mit einem pKa von gleich oder größer als 2,8, durchgeführt wird.

6. Verfahren nach Anspruch 5, wobei die Reaktion zwischen dem organischen Carbonat und dem aromatischen Amin in Gegenwart von wasserfreiem Zinkacetat oder Zinkacetatdihydrat durchgeführt wird.

7. Verfahren nach einem der vorhergehenden Ansprüche, wobei die Reaktion in Gegenwart von organischem Carbonat, das alleine als Lösungsmittel dient, durchgeführt wird.

8. Verfahren nach einem der vorhergehenden Ansprüche, wobei die Reaktion von der Entfernung des Alkohols, während sich dieser bildet, begleitet ist.

9. Verfahren nach einem der vorhergehenden Ansprüche, wobei die Reaktion ausgehend von einem Amin mit der Formel R-(NH₂)ₙ durchgeführt wird, wobei n 1 oder 2 ist und R einen Arylrest, ausgewählt aus monovalenten und bivalenten Resten von Benzol, Toluol, Naphthalen, Diphenyl und Methylendiphenyl, darstellt.

10. Verfahren nach einem der vorhergehenden Ansprüche, wobei die Reaktion ausgehend von einem Amin, vorzugsweise ausgewählt aus 2,4-Diaminotoluol, 2,6-Diaminotoluol, Mischungen von 2,4-Diaminotoluol und 2,6-Diaminotoluol, Anilin, Toluidin, 3,5-Diaminotoluol, 4,4-Methylendianilin, 2,4-Methylendianilin, 2,2-Methylendianilin, und Mischungen aus zwei oder mehreren von 4,4-Methylendianilin, 2,4-Methylendianilin und 2,2-Methylendianilin, durchgeführt wird.

## Revendications

1. Procédé de préparation d'uréthanes aromatiques comprenant la mise en réaction de carbonates organiques avec des amines aromatiques dans des réacteurs fonctionnant en continu et l'alimentation d'une quantité totale d'amines aromatiques en un pourcentage par rapport au poids du mélange total compris dans la plage allant de 4 à 30.

2. Procédé de préparation d'uréthanes aromatiques selon la revendication précédente, dans lequel la réaction entre les carbonates organiques et les amines aromatiques est réalisée dans des réacteurs en continu, la concentration de l'amine aromatique étant maintenue à 0,1 à 5 % en poids par rapport au mélange total.

3. Procédé de préparation d'uréthanes aromatiques selon la revendication précédente, dans lequel la concentration de l'amine aromatique est de préférence maintenue à 0,1 à 2,5 % par rapport au mélange total.

4. Procédé de préparation d'uréthanes aromatiques comprenant la mise en réaction de carbonates organiques avec des amines aromatiques selon l'une quelconque des revendications précédentes, **caractérisé en ce que** la réaction est réalisée dans des réacteurs choisis parmi les réacteurs de type CSTR ou colonne réactive.

5. Procédé de préparation d'uréthanes aromatiques comprenant la mise en réaction de carbonates organiques avec des amines aromatiques selon l'une quelconque des revendications précédentes, **caractérisé en ce que** la réaction est réalisée en présence d'un catalyseur choisi parmi les acides de Lewis de pKa supérieur ou égal à 2,8.

6. Procédé de préparation d'uréthanes aromatiques selon la revendication précédente, **caractérise en ce que** la réaction entre le carbonate organique et l'amine aromatique est de préférence réalisée en présence d'acétate de zinc anhydre ou d'acétate de zinc dihydraté.

7. Procédé de préparation d'uréthanes aromatiques comprenant la mise en réaction de carbonates organiques avec des amines aromatiques selon l'une quelconque des revendications précédentes, **caractérisé en ce que** la réaction est réalisée en présence de carbonate organique seul agissant comme solvant.

8. Procédé de préparation d'uréthanes aromatiques comprenant la mise en réaction de carbonates organiques avec des amines aromatiques selon l'une quelconque des revendications précédentes, dans lequel la réaction est accompagnée par l'élimination de l'alcool dès sa formation.

9. Procédé de préparation d'uréthanes aromatiques comprenant la mise en réaction de carbonates organiques avec des amines aromatiques selon l'une quelconque des revendications précédentes, **caractérisé en ce que** la réaction est réalisée en commençant avec une amine ayant la formule R-(NH₂)ₙ dans laquelle n vaut 1 ou 2, R représente un radical aryle choisi parmi les radicaux monovalents et bivalents de benzène, toluène, naphtalène, diphényle, et méthylène-diphényle.

10. Procédé de préparation d'uréthanes aromatiques comprenant la mise en réaction de carbonates organiques avec des amines aromatiques selon l'une quelconque des revendications précédentes, **caractérisé en ce que** la réaction est réalisée en commençant avec une amine de préférence choisie parmi le 2,4-diaminotoluène, le 2,6-diaminotoluène ou leurs mélanges, l'aniline, la toluidine, la 3,5-diaminotoluène, la 4,4-méthylènedianiline, la 2,4-méthylènedianiline, la 2,2-méthylènedianiline ou des mélanges de ces isomères.
